# EUROPÄISCHE PATENTSCHRIFT

(11) **EP 0 990 017 B1**
(45) Veröffentlichungstag und Bekanntmachung des Hinweises auf die Patenterteilung: **08.08.2001**
(21) Anmeldenummer: 98936332.0
(22) Anmeldetag: 12.06.1998
(51) Int. Cl.: C10L 3/10, B01D 53/14

(54) **VERFAHREN UND ANORDNUNG ZUM TROCKNEN EINES GASES**
METHOD AND DEVICE FOR DRYING A GAS
PROCEDE ET DISPOSITIF POUR LE SECHAGE D'UN GAZ

(30) Priorität: 20.06.1997 DE 19726210; 16.05.1998 DE 19822165
(43) Veröffentlichungstag der Anmeldung: 05.04.2000
(73) Patentinhaber: RUHRGAS AKTIENGESELLSCHAFT, 45138 Essen (DE)
(72) Erfinder: FORSTER, Ruediger, D-45894 Gelsenkirchen (DE)
(74) Vertreter: Harlacher, Mechthild, Dipl.-Ing.
(86) Internationale Anmeldenummer: EP9803550
(87) Internationale Veröffentlichungsnummer: WO9859021

(56) Entgegenhaltungen:
- US-A- 5 490 873
- CHEMICAL ABSTRACTS, vol. 79, no. 4, 30. Juli 1973 Columbus, Ohio, US; abstract no. 20814, MARTYNEK, MIECZYSLAW ET AL: "Diethylene glycol desalting with ion exchangers" XP002085145 & NAFTA (KATOWICE) (1972), 28(12), 554-5 CODEN: NAFPAB,1972,

## Beschreibung

Die Erfindung bezieht sich auf ein Verfahren und eine Anordnung zum Trocknen eines Gases, insbesondere von Erdgas, unter Verwendung eines Glykols sowie Maßnahmen zum Reinigen von in einer Gastrocknungsanlage verunreinigtem Glykol.

Glykole, insbesondere Triethylenglykol, werden in großem Umfang zum Trocknen von Gasen, insbesondere von Erdgas, verwendet. Beim Transportieren von Erdgas unter hohem Druck darf der Wasserdampfgehalt einen druckabhängigen Schwellenwert nicht überschreiten. Durch kondensiertes Wasser können Rohrleitungskorrosionen auftreten. Auch wäre die Bildung von Gashydraten möglich.

Um den Wassergehalt des Gases zu reduzieren, wird es in Trocknungsanlagen mit Glykol als Absorptionsmittel in innigen Kontakt gebracht. Beispielsweise wirken zu trocknendes Gas und Glykol in einem Absorber im Gegenstrom aufeinander ein. Dabei nimmt das Glykol Feuchtigkeit aus dem Gas, aber auch Verunreinigungen auf. Das wasserbeladene und verunreinigte Glykol aus dem Absorber wird anschließend regeneriert, wobei Wasser aus der Glykol-Wasser-Mischung ausgetrieben wird.

Aus der US-A-5 490 873 ist ein Verfahren bekannt, bei dem die in der Regeneration freigesetzten Dämpfe partiell kondensiert und anschließend einem Separator zugeführt werden. In diesem werden drei Phasen, und zwar eine Gasphase, eine flüssige Kohlenwasserstoffphase und eine Wasserphase, voneinander getrennt. Die Gasphase wird komprimiert und dem Prozeß zugeführt. Die flüssige Kohlenwasserstoffphase wird der Verwertung und die Wasserphase der Entsorgung zugeführt.

Das getrocknete Glykol aus der Regeneration wird erneut dem Absorber zugeführt. In diesem Kreislauf reichert sich das Glykol zunehmend mit Verunreinigungen an. Zu diesen Verunreinigungen gehören u.a. Salze, Partikel, Zersetzungsprodukte des Glykols sowie organische Verunreinigungen, die aus dem zu trocknenden Gas aufgenommen worden sind. Ein Teil der Zersetzungsprodukte des Glykols und der aus dem zu trocknenden Gas aufgenommenen Verunreinigungen sind organische Säuren, die zu einem Abfall des pH-Werts führen, was eine erhöhte Anlagenkorrosion bewirken kann. Außerdem können diese und andere organische Verunreinigungen und Zersetzungsprodukte zur Schaumbildung führen, was die Trocknungsfähigkeit des Glykols vermindert und zu hohen Glykolverlusten führen kann.

Deshalb sind bei bekannten Anlagen ein regelmäßiger Austausch oder eine Reinigung des Glykois erforderlich. Die Reinigung des aus der Trocknungsanlage abgeführten Glykols erfolgt üblicherweise durch Destillation.

Aufgabe der Erfindung ist es, ein Gastrocknungsverfahren zu schaffen, das eine wirtschaftlichere Verwendung des eingesetzten Glykols, insbesondere eine längere Nutzungsdauer des Glykols gestattet

Diese Aufgabe wird erfindungsgemäß durch ein Verfahren mit den Merkmalen des Patentanspruchs 1 gelöst. Das zu trocknende Gas wird einem Absorber zugeführt, in dem Absorber in innigen Kontakt mit einem Glykol gebracht und anschließend aus dem Absorber abgeführt. Das wasserbeladene, verunreinigte Glykol wird aus dem Absorber abgeführt und regeneriert. Wenigstens ein Teil des aus dem Absorber abgeführten Glykols wird vor oder auch nach seiner Regeneration gereinigt, indem das Glykol mit mindestens der halben Menge Wasser gemischt, die Mischung auf eine Temperatur oberhalb ihres Trübungspunktes gebracht und dort für eine vorgegebene Zeitdauer gehalten wird, wobei Verunreinigungen ausflocken, anschließend die ausgeflockten Verunreinigungen entfernt werden und das Wasser aus der Mischung durch Erhitzen ausgetrieben wird. Es wurde überraschenderweise festgestellt, daß ein wesentlicher Anteil der Verunreinigungen des Glykols auf dieser Weise in die oberhalb des Trübungspunktes entstehenden Ausflockungen aufgenommen wird, so daß eine anschließende Abtrennung zu einer ausreichenden Reinigung des Glykols führt. Insbesondere werden bei dem Verfahren Öl, Schwarzschlamm und andere Oxidationsprodukte aus dem Glykol entfernt. Das erfindungsgemäße Verfahren stellt ein sehr wirtschaftliches Trocknungsverfahren dar, da es eine umweltverträgliche und unaufwendige Reinigung des Glykols integriert.

Bei einem Ausführungsbeispiel des erfindungsgemäßen Verfahrens wird das aus dem Absorber abgeführte Glykol vor dem Regenerieren gereinigt. Dabei wird das bei der Reinigung zugemischte Wasser zusammen mit dem im Absorber aufgenommenen Wasser in einem Regenerierschritt durch Erhitzen ausgetrieben. Bei diesem Hauptstrom-Verfahren wird stets die gesamte zu regenerierende, d.h. vom Wasser zu befreiende, Glykolmenge vorab der Reinigung unterzogen. Da bei diesem Verfahren stets der gesamten, im Umlauf befindlichen Glykolmenge eine entsprechende Wassermenge zugemischt werden muß, ist für die Trennung von Wasser und Glykol im Regenerierschritt ein hoher Energieaufwand erforderlich.

Bei einer weiteren Ausführungsform des erfindungsgemäßen Verfahrens wird ein Teil des zuvor regenerierten Glykols gereinigt. Das Glykol wird zunächst in der herkömmlichen Weise durch Austreiben des Wassers regeneriert. Das regenerierte, aber noch verunreinigte Glykol wird anschließend zum Teil einem Reinigungsprozeß zugeführt. Der verbleibende Teil des regenerierten Glykols wird dem Absorber zugeführt.

Vorzugsweise wird nach Entfernen der ausgeflockten Verunreinigungen die Glykol-Wasser-Mischung erneut mit dem zu regenerierenden aus dem Absorber abgeführten Glykol gemischt und anschließend regeneriert. Dieses Nebenstromverfahren erlaubt es, jeweils nur einen Teil des im Absorber-Kreislauf befindlichen Glykols zu reinigen, so daß die zum Austreiben des aus dem Gas aufgenommenen Wassers und des bei der Reinigung zugemischten Wassers im Regenerierschritt erforderliche Energie reduziert werden kann.

Zum Entfernen der ausgeflockten Verunreinigungen wird die Glykol-Wasser-Mischung vorzugsweise filtriert und/oder zentrifugiert. Neben diesen schnellen Trennverfahren sind verfahrenstechnische Alternativen wie Absetzenlassen oder Flokkulation möglich.

Bei einer vorteilhaften Weiterbildung des erfindungsgemäßen Verfahrens wird die Glykol-Wasser-Mischung vor dem Erwärmen über einen ersten Anionenaustauscher geleitet.

Vorteilhafterweise wird die Glykol-Wasser-Mischung beim Reinigen auf eine Temperatur von etwa 40°C bis 90°C gebracht und dort für eine Zeitdauer zwischen 2 und 30 Minuten gehalten. Temperatur, erforderliche Zeitdauer und Wasseranteil in der Mischung stehen in einem wechselseitigen Zusammenhang. Ein höherer Wasseranteil führt zu einer schnelleren Trübung bzw. Ausflockung der Verunreinigungen; andererseits wird durch einen höheren Wasseranteil die zum Austreiben des Wassers aus der Mischung erforderliche Zeit und Energiemenge erhöht. Vorzugsweise wird das verunreinigte Glykol mit Wasser etwa im Verhältnis 1:1 gemischt.

In Weiterbildung des erfindungsgemäßen Verfahrens wird als Trockenmittel Triethylenglykol (TEG) verwendet. Dabei wird das verunreinigte Triethylenglykol beim Reinigen mit Wasser im Verhältnis 1:1 gemischt und die Triethylenglykol-Wasser-Mischung auf eine Temperatur von etwa 75°C bis 85°C gebracht und dort für eine Zeitdauer zwischen 5 und 20 Minuten gehalten. Die Temperatur liegt über dem sogenannten "Cloud Point". Vorzugsweise wird die Mischung aus Wasser und TEG vor dem Erwärmen über einen Anionenaustauscher geführt.

Die der Erfindung zugrunde liegende Aufgabe wird darüber hinaus durch ein Reinigungsverfahren mit den Merkmalen des Patentanspruchs 7 gelöst.

Aus der Gastrocknungsanlage wird verunreinigtes Glykol abgeführt. Es kann sowohl getrocknetes (regeneriertes) Glykol als auch wasserbeladenes Glykol aus dem Kreislauf der Gastrocknungsanlage abgeführt werden. Das abgeführte Glykol wird mit mindestens der halben Menge Wasser vermischt. Die Glykol-Wasser-Mischung wird anschließend über einen ersten Anionenaustauscher geführt, wobei aus der Mischung anionische Tenside entfernt werden. Dabei wird der Trübungspunkt unter die Temperatur der Mischung abgesenkt, wodurch Verunreinigungen ausflocken. Vorzugsweise wird das abgeführte Glykol bei einer Temperatur aus der Gastrocknungsanlage abgeführt und der Reinigung zugeführt, die ausreichend hoch ist, so daß die Temperatur der Glykol-Wasser-Mischung nach dem Austauschen der anionischen Tenside bereits über dem Trübungspunkt liegt, ohne daß ein zusätzliches Aufheizen der Glykol-Wasser-Mischung vor oder nach dem Durchgang durch den lonenaustauscher erforderlich wird.

Die ausgeflockten Verunreinigungen werden anschließend entfernt. Die gereinigte Glykol-Wasser-Mischung wird in eine Regenerationsstufe der Gastrocknungsanlage zurückgeführt, in der das Wasser ausgetrieben wird.

Das erfindungsgemäße Verfahren führt zu einer ausgezeichneten Reinigung des Glykols; ein wesentlicher Anteil der Verunreinigungen des Glykols wird in die abgetrennten Ausflockungen aufgenommen. Insbesondere werden bei dem Verfahren Öle, Schwarzschlamm und Oxidationsprodukte aus dem Glykol entfernt. Das erfindungsgemäße Verfahren ist sehr wirtschaftlich und umweltverträglich.

Auch gegenüber dem aus der deutschen Patentanmeldung 197 26 210.4 bekannten Reinigungsverfahren hat das erfindungsgemäße Verfahren eine Reihe von Vorteilen. Das Verfahren ist wirtschaftlicher und erleichtert den kontinuierlichen Betrieb einer Reinigungsanlage, da das Glykol-Wasser-Gemisch nach dem Zumischen des Wassers nicht so stark erwärmt werden muß oder das Erwärmen ganz entfallen kann. Dies wird durch eine Absenkung des Trübungspunktes bewirkt, der folgender Mechanismus zugrunde liegt: Sehr häufig ist das Glykol von Gastrocknungsanlagen durch hochsiedende Fette und Öle verunreinigt. Diese Stoffe liegen im Glykol emulgiert vor. Als Emulgatoren wirken neben nichtionischen Fettalkoholethoxylaten und Zuckertensiden langkettige anionische Carbonsäuren (Fettsäuren). Bei dem üblicherweise durch Zugabe von Aminen auf Werte > 7 eingestellten pH-Wert liegen diese Substanzen im Glykol dissoziiert, d.h. geladen vor. Somit tragen auch die die Verunreinigungen enthaltenen Micellen der Emulsion negative Ladungen, wodurch sie sich abstoßen, was einer Spaltung der Emulsion entgegenwirkt. Nachdem die anionischen Tenside durch den ersten lonenaustauscher aus der Glykol-Wasser-Mischung entfernt worden sind, agglomerieren die nun ungeladenen Micellen und können leichter abgeschieden werden. Dies äußerst sich in einer Absenkung des Trübungspunktes, d.h. der Temperatur, bei der Verunreinigungen ausflocken.

Der erste lonenaustauscher wird vorzugsweise mit Anionen einer starken Säure, insbesondere mit Chlorid-lonen, beladen. Das lonenaustauscherharz gibt dosiert Chlorid-lonen an das Glykol-Wasser-Gemisch ab, wobei aus dem Gemisch (unter anderem) die oberflächenaktiv wirkenden Carbonsäure-Anionen aufgenommen werden. Der lonenaustauscher kann durch Rückspülen mit einer Kochsalzlösung regeneriert werden. Durch die Abgabe von Chlorid-lonen an das Glykol-Wasser-Gemisch wird zusätzlich die lonenstärke der Mischung erhöht, wodurch die Löslichkeit der die Verunreinigungen enthaltenden Micellen weiter verringert wird. Dies führt zu einer weiteren Absenkung der Trübungspunkt-Temperatur, bei der sich die micellare Phase von der Lösung abtrennt. Ein Erwärmen des Glykol-Wasser-Gemisches zum Erzielen der Ausflockung der Verunreinigungen ist unter diesen Bedingungen nicht mehr erforderlich.

Bei einer vorteilhaften Weiterbildung des erfindungsgemäßen Verfahrens wird die gereinigte Glykol-Wasser-Mischung vor dem Zurückführen in die Regenerationsstufe der Gastrocknungsanlage durch einen basischen, mit Hydroxid-Ionen beladenen zweiten lonenaustauscher geleitet, wobei die im ersten lonenaustauscher der Mischung zugeführten Anionen zumindest teilweise gegen Hydroxid-Ionen ausgetauscht werden. Durch das Austauschen dieser Säureanionen gegen Hydroxid-lonen wird eine Korrosion der Trocknungsanlage durch die zugeführten Säureanionen, insbesondere die Chlorid-lonen, vermieden. Vorzugsweise wird ein stark basischer lonenaustauscher verwendet. Der Austausch der Säureanionen gegen Hydroxid-Anionen wird auch als Entsalzung des Glykols bezeichnet. Der zweite lonenaustauscher kann z.B. mit Natronlauge regeneriert werden.

In vorteilhafter Weiterbildung der Erfindung wird die Glykol-Wasser-Mischung zur Entfernung der ausgeflockten Verunreinigungen durch ein Sand- oder Kiesbettfilter geführt. Vorzugsweise wird ein mehrstufiges Filter verwendet, wobei die Mischung zunächst durch eine gröbere und anschließend eine feinere Sandfraktion geleitet wird. Mit zunehmender Beladung des Filters mit ausgeflockten Verunreinigungen kommt es zu einem Druckanstieg. Dann müssen die Sandfilter durch Rückspülen regeneriert werden. Dazu kann ein Teil des gereinigten Glykol-Wasser-Gemisches verwendet werden.

Die der Erfindung zugrunde liegende Aufgabe wird darüber hinaus durch eine Anordnung mit den Merkmalen des Patentanspruchs 11 gelöst.

Die Anordnung zum Trocknen eines Gases, insbesondere von Erdgas, weist einen Absorber auf, in dem das zu trocknende Gas und ein Glykol aufeinander einwirken, wobei das Glykol Wasser aufnimmt. Mit dem Absorber ist eine Einrichtung zum Abführen des wasserbeladenen Glykols gekoppelt, die das Glykol einem Reboiler zum Regenerieren des Glykols zuführt. Mit dem Reboiler ist eine Einrichtung zum Rückführen des regenerierten Glykols zum Absorber verbunden. Mit der Einrichtung zum Rückführen ist bei der erfindungsgemäßen Einrichtung eine Mischeinrichtung zum Abführen eines Anteils des regenerierten Glykols und zum Zumischen einer vorgegebenen Wassermenge verbunden. Mit der Mischeinrichtung ist eine Absetzkammer verbunden, die die Mischung aus Glykol und Wasser aufnimmt, wobei die Mischung eine vorgegebene Zeitdauer in der Absetzkammer verweilen kann, so daß Verunreinigungen in der Absetzkammer ausflocken. Eine Trenneinrichtung ist derart mit der Absetzkammer verbunden, daß die Mischung mit den ausgeflockten Verunreinigungen zugeführt werden kann, wobei die Trenneinrichtung die ausgeflockten Verunreinigungen abtrennt. Die Trenneinrichtung ist mit dem Reboiler verbunden und führt diesem die gereinigte Glykol-Wasser-Mischung zu. Diese erfindungsgemäße Anordnung führt das energetisch günstigere Nebenstrom-Verfahren zum Trocknen des Gases aus, welches oben beschrieben wurde.

Bei einem Ausführungsbeispiel weist die Einrichtung zum Rückführen des regenerierten Glykols zum Absorber einen Vorratsbehälter auf, wobei die Mischeinrichtung mit dem Vorratsbehälter, vorzugsweise über eine Pumpe, verbunden ist. Als Trenneinrichtung wird ein Feinfilter verwendet.

Vorzugsweise ist zwischen der Mischeinrichtung und der Absetzkammer ein Anionenaustauscher zur Vorreinigung der Glykol-Wasser-Mischung eingebunden.

Nach einem weiteren Merkmal der Erfindung kann zwischen der Mischeinrichtung und der Trenneinrichtung ein lonentauscher eingebunden sein, in dem aus der Glykol-Wasser-Mischung anionische Tenside entfernt werden.

Die der Erfindung zugrunde liegende Aufgabe wird darüber hinaus durch eine Anordnung mit den Merkmalen des Anspruchs 14 gelöst.

Die Anordnung zum Reinigen von in einer Gastrocknungsanlage verunreinigtem Glykol weist eine Mischeinrichtung, einen ersten lonenaustauscher, eine Filtereinrichtung und eine Regenerationsstufe auf. Die Mischeinrichtung dient zum Mischen von aus der Gastrocknungsanlage abgeführtem Glykol mit Wasser, der erste lonenaustauscher zum Entfernen anionischer Tenside aus der Glykol-Wasser-Mischung, die Filtereinrichtung zum Entfernen ausgeflockter Verunreinigungen und die Regenerationsstufe zum Austreiben des Wassers aus der Mischung.

Bei einer bevorzugten Ausführungsform ist der erste lonenaustauscher mit Anionen einer starken Säure, vorzugsweise mit Chlorid-lonen beladen.

Eine vorteilhafte Weiterbildung der Erfindung ist dadurch gekennzeichnet, daß der Filtereinrichtung ein basischer, mit Hydroxid-lonen beladener zweiter lonenaustauscher zum Austauschen zumindest eines Teils der im ersten lonenaustauscher der Glykol-Wasser-Mischung zugeführten Anionen gegen Hydroxid-Anionen nachgeordnet ist. Dies verhindert einen zusätzlichen Korrosionsangriff der Säureanionen.

Die Filtereinrichtung kann ein mehrstufiges Sand- oder Kiesbettfilter aufweisen. Dabei sind gröbere und stromab feinere Sandfraktionen hintereinander angeordnet. Zusätzlich kann ein Aktivkohlefilter vorgesehen sein. Sowohl der erste und der zweite Anionenaustauscher als auch die Filtereinrichtung sind mit Einrichtungen (Rohrleitungen, Ventilen und entsprechenden Steuereinrichtungen) ausgerüstet, die ein Rückspülen zur Regenerierung bzw. Reinigung gestatten.

Eine bevorzugte Ausführungsform der erfindungsgemäßen Anordnung ist dadurch gekennzeichnet, daß die Mischeinrichtung über eine Leitung derart mit einer das Glykol einem Absorber zuführenden Pumpe der Gastrocknungsanlage verbunden ist, daß in die Mischeinrichtung ein Teil des aus der Pumpe austretenden Glykols gedrückt wird. Durch eine derartige Ankopplung des Reinigungskreislaufs kann auf eine zusätzliche Pumpe im Reinigungskreislauf verzichtet werden. Außerdem liegt das Glykol an dieser Stelle des Kreislaufs der Gastrocknungsanlage in einer ausreichend hohen Temperatur vor, so daß auf eine zusätzliche Erwärmung zur Überschreitung des Trübungspunktes verzichtet werden kann.

Im folgenden wird die Erfindung anhand eines in der Zeichnung dargestellten Ausführungsbeispiels näher erläutert.
- Fig. 1: zeigt ein bevorzugtes Ausführungsbeispiel der erfindungsmäßigen Anordnung zum Trocknen eines Gases.
- Fig. 2: zeigt eine Gastrocknungsanlage mit einer erfindungsgemäßen Anordnung zum Reinigen des in der Gastrocknungsanlage verunreinigten Glykols.
- Fig. 3: zeigt eine Gastrocknungsanlage mit einem alternativen Ausführungsbeispiel der erfindungsgemäßen Anordnung zum Reinigen des Glykols in bevorzugter Ankopplung an den Glykol-Kreislauf der Gastrocknungsanlage.

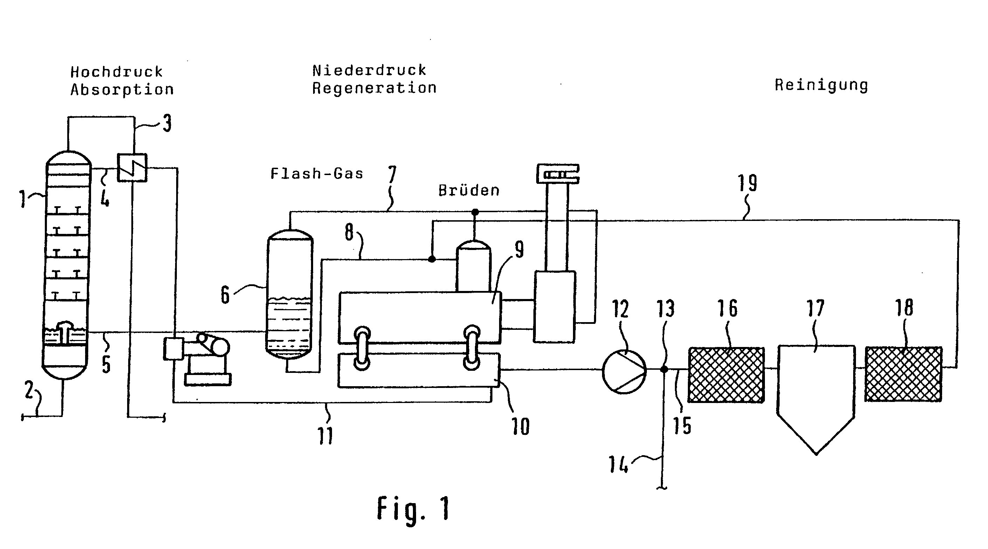

In Fig. 1 wird das zu trocknende Gas einem Ende eines Absorbers 1 über eine Leitung 2 zugeführt. An einem gegenüberliegenden Ende des Absorbers 1 wird das getrocknete Gas über eine Leitung 3 abgeführt. Um das Gegenstromprinzip zu verwirklichen, wird das regenerierte, wasserfreie Glykol über eine Leitung 4 an dem Ende des Absorbers 1 zugeführt, an dem das getrocknete Gas abgeführt wird. In dem Absorber 1 nimmt das im Gegenstrom geführte Glykol das Wasser aus dem Gas sowie Verunreinigungen auf. Das mit Wasser beladene und verunreinigte Glykol wird über eine Leitung 5 aus dem Absorber abgeführt und in einen Flash-Tank 6 eingeleitet.

In dem Flash-Tank verweilt das beladene Glykol und gibt dabei Gas ab. Das entweichende Gas wird über eine Leitung 7 abgeführt. Das mit Wasser beladene Glykol wird über Leitung 8 dem Arbeitsbehälter des Reboilers 9 zugeführt. Im Reboiler 9 wird das wasserbeladene Glykol (die Glykol-Wasser-Mischung) erhitzt, wobei das Wasser verdampft. Das verbleibende, regenerierte Glykol gelangt aus dem Arbeitsbehälter des Reboilers 9 in einen Vorratsbehälter 10. Aus dem Vorratsbehälter 10 wird das regenerierte Glykol über die Leitungen 11 und 4 erneut dem Hochdruck-Absorber 1 zugeführt.

Parallel zu diesem Verfahren der Wasserabsorption und Glykol-Regenerierung wird das Glykol in einem Nebenstrom-Verfahren gereinigt.

Dazu wird ein Teil des regenerierten Glykols aus dem Vorratsbehälter mittels der Pumpe 12 einer Mischeinrichtung 13 zugeführt. Die Mischeinrichtung 13 ist mit einer Wasserzuleitung 14 verbunden. Die Mischeinrichtung 13 mischt das über die Pumpe 12 zugeführte Glykol und das über Leitung 14 zugeführte Wasser in einem einstellbaren Mengenverhältnis.

Die Glykol-Wasser-Mischung gelangt über Leitung 15 in einen Anionenaustauscher 16, wo sie vorgereinigt wird. Der Anionenaustauscher enthält beispielsweise ein Polymerharz vom Typ Dowex 1 x 8. Aus dem Anionenaustauscher gelangt die vorgereinigte Glykol-Wasser-Mischung in die Absetzkammer 17, wo sie eine vorgegebene Zeitdauer bei einer vorgegebenen Temperatur verweilt. Dabei flocken Verunreinigungen des Glykols aus. Die Vorreinigung im Anionenaustauscher beschleunigt das Ausflocken der Verunreinigungen auch bei geringeren Wassergehalten. Nach dem Ausflocken der Verunreinigungen wird die Mischung aus der Absetzkammer 17 durch ein Feinfilter 18 hindurchgepumpt, wobei die ausgeflockten Verunreinigungen entfernt bzw. abgetrennt werden.

Die auf diese Weise gereinigte Glykol-Wasser-Mischung wird über Leitung 19 erneut dem Reboiler zugeführt. Dort wird die Mischung zusammen mit dem über die Leitung 8 zugeführten wasserbeladenen Glykol erhitzt, wobei sowohl das aus dem Gas absorbierte Wasser als auch das von der Mischeinrichtung 13 zugemischte Wasser verdampft werden.

Bei einer bevorzugten Ausführungsform des erfindungsgemäßen Verfahrens wird verunreinigtes TEG gereinigt, indem es mit etwa der gleichen Menge Wasser gemischt wird. Die Mischung wird anschließend für etwa 15 Minuten auf eine Temperatur zwischen 75 und 85°C gebracht. Es scheidet sich eine micellare Phase ab, die einen überwiegenden Anteil der Verunreinigungen enthält. Anschließend wird die Mischung filtriert und das Wasser in einem Reboiler bei Temperaturen zwischen 185 und 205°C ausgetrieben.

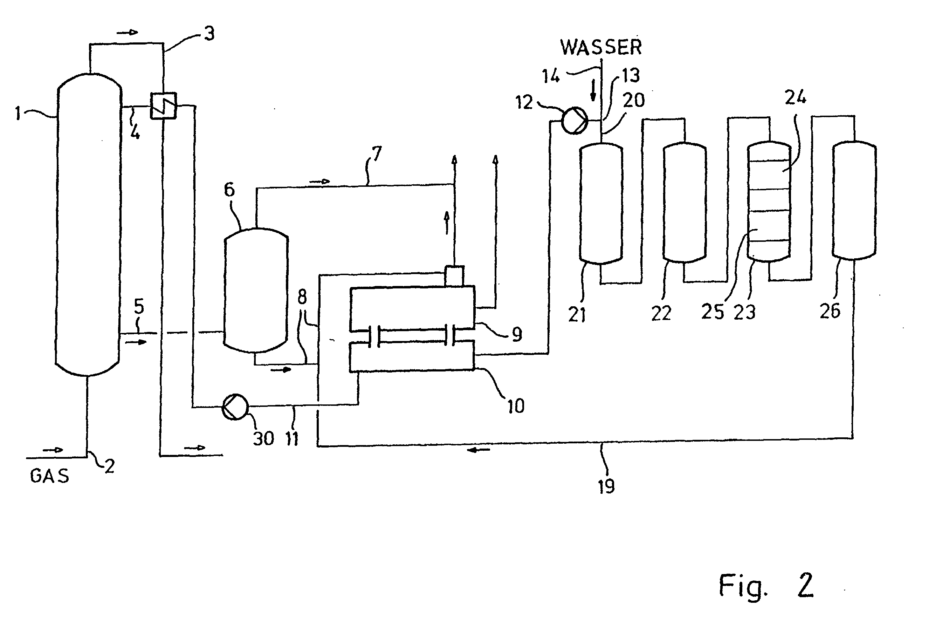

In der Gastrocknungsanlage gemäß Fig. 2 wird das zu trocknende Gas einem Ende eines Absorbers 1 über eine Leitung 2 zugeführt. An einem gegenüberliegenden Ende des Absorbers 1 wird das getrocknete Gas über die Leitung 3 abgeführt. Im Gegenstrom wird regeneriertes, wasserfreies Glykol über eine Leitung 4 an dem Ende des Absorbers 1 zugeführt, an dem das getrocknete Gas abgeführt wird. In dem Absorber nimmt das im Gegenstrom geführte Glykol das Wasser aus dem Gas sowie Verunreinigungen auf. Das mit Wasser beladene und verunreinigte Glykol wird über eine Leitung 5 aus dem Absorber abgeführt und in einen Flash-Tank 6 , eingeleitet. In dem Flash-Tank verweilt das wasserbeladene Glykol und gibt dabei Gas ab. Das entweichende Gas wird über eine Leitung 7 abgeführt. Das wasserbeladene Glykol wird über Leitung 8 dem Arbeitsbehälter eines Reboilers 9 zugeführt. Im Reboiler 9 wird das wasserbeladene Glykol (die Glykol-Wasser-Mischung) erhitzt, wobei das Wasser verdampft. Das verbleibende, regenerierte Glykol gelangt aus dem Arbeitsbehälter des Reboilers 9 in einen Vorratsbehälter 10. Aus dem Vorratsbehälter 10 wird das regenerierte, getrocknete Glykol über die Leitung 11, die Pumpe 30 und die Leitung 4 erneut dem Absorber 1 zugeführt.

Parallel zu diesem Verfahren der Wasserabsorption und Glykol-Regenerierung wird das Glykol in einem Nebenstrom-Verfahren gereinigt. Der Reinigungskreislauf beginnt bei dem in Fig. 1 dargestellten Ausführungsbeispiel im Vorratsbehälter 10. Ein Teil des regenerierten Glykols wird aus dem Vorratsbehälter mittels der Pumpe 12 einer Mischeinrichtung 13 zugeführt. Die Mischeinrichtung 13 ist mit einerWasserzuleitung 14 verbunden. Die Mischeinrichtung 13 mischt das über die Pumpe 12 zugeführte Glykol und das über Leitung 14 zugeführte Wasser in einem einstellbaren Mengenverhältnis. Vorzugsweise wird das über Pumpe 12 zugeführte Glykol mit mindestens derselben Menge Wasser vermischt. Dabei wurde festgestellt, daß ein höherer Wasseranteil zu einer schnelleren Trübung bzw. Ausflockung der Verunreinigungen führt. Andererseits wird durch einen höheren Wasseranteil die bei der Regenerierung zum Austreiben des Wassers aus der Mischung erforderliche Zeit und Energiemenge erhöht. Die Bestimmung der erforderlichen Wassermenge stellt demzufolge eine Optimierungsaufgabe dar; vorzugsweise wird das Glykol mit dem Wasser etwa im Verhältnis 1:1 gemischt.

Die Glykol-Wasser-Mischung gelangt über Leitung 20 in den ersten Anionenaustauscher 21, der mit Chlorid-Anionen beladen ist. In dem ersten Anionenaustauscher wird ein großer Teil der anionischen Tenside (überwiegend Carbonsäureanionen) der Glykol-Wasser-Mischung gegen Chlorid-lonen ausgetauscht. Dabei befindet sich die Glykol-Wasser-Mischung auf einer Temperatur, die oberhalb des Trübungspunktes liegt, so daß sich die micellare Phase abtrennt. Nachdem die anionischen Tenside entfernt worden sind, agglomerieren die auf diese Weise entladenen Micellen. Der Trübungspunkt wird zusätzlich dadurch verringert, daß durch die Abgabe von Chlorid-lonen an die Mischung deren lonenstärke erhöht und somit die Löslichkeit der Micellen weiter verringert wird. Sollte die Temperatur der Glykol-Wasser-Mischung im Anionenaustauscher 21 unterhalb des Trübungspunktes (Cloud-Point) liegen, so kann die Mischung im oder nach Verlassen des lonenaustauschers 21 ggf. erwärmt werden.

Die Glykol-Wasser-Mischung mit ausgeflockten Verunreinigungen wird anschließend durch ein Kiesbett- oder Sandbettfilter 22 geleitet. Die ausgeflockten Verunreinigungen werden dort abgetrennt. Bei dem in Fig. 1 dargestellten Ausführungsbeispiel ist dem ersten Sandbettfilter 22 ein zweites Filter 23 nachgeschaltet, wobei das zweite Filter 23 ein Aktivkohlefilter 24 und ein feines Sandbettfilter 25 aufweist.

Nach Entfernen der ausgeflockten Verunreinigungen wird die Glykol-Wasser-Mischung einem zweiten Anionenaustauscher 26 zugeführt. Dieser stark basische, mit Hydroxid-lonen beladene zweite Anionenaustauscher 26 tauscht die durch den ersten Anionenaustauscher 21 in die Glykol-Wasser-Mischung eingebrachten Chlorid-lonen gegen Hydroxid-lonen aus. Dies wird als Entsalzung bezeichnet. Die Chlorid-Ionen müssen aus dem Glykol entfernt werden, da sie eine Korrosion der Reinigungs- und Gastrocknungsanlage bewirken können. Der zweite Anionenaustauscher kann mit einer Natronlauge regeneriert werden.

Im Anschluß an die Entsalzung wird die Glykol-Wasser-Mischung über Leitung 19 in die Regenerationsstufe der Gastrocknungsanlage zurückgeführt, indem sie in den Arbeitsbehälter des Reboilers 9 eingeleitet wird. Dort wird das Wasser aus der Mischung ausgetrieben. Das regenerierte, getrocknete Glykol gelangt wieder in den Vorratsbehälter 10.

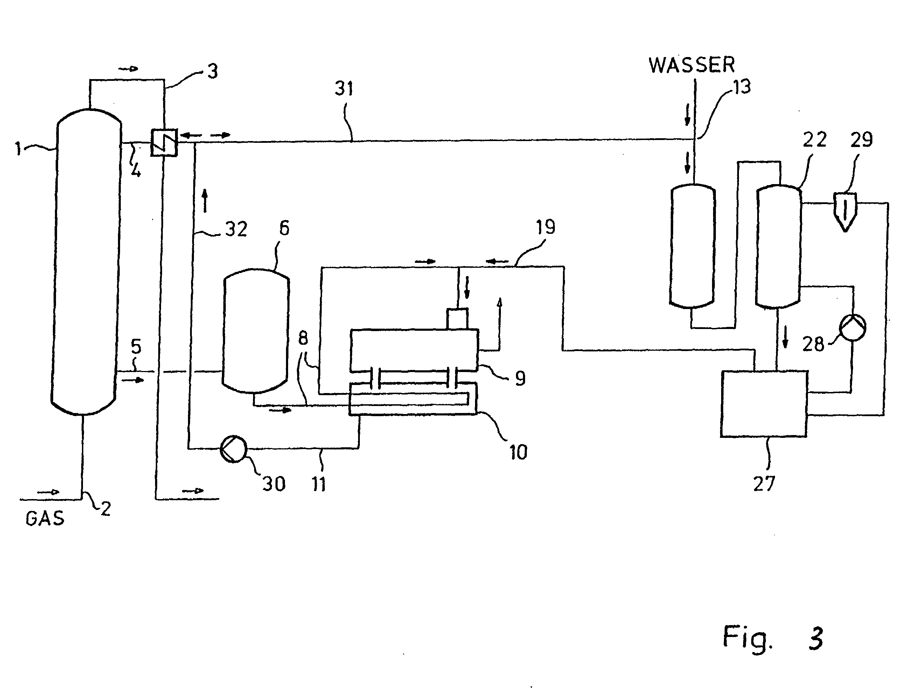

Fig. 3 zeigt eine Gastrocknungsanlage mit einem weiteren Ausführungsbeispiel der erfindungsgemäßen Anordnung zum Reinigen des Glykols. Die Gastrocknungsanlage entspricht im wesentlichen der in Fig. 1 dargestellten. Die Glykolreinigung erfolgt auch hier im Nebenstromverfahren. Im Unterschied zu dem in Fig. 1 darstellten Ausführungsbeispiel wird bei der Anlage gemäß Fig. 2 regeneriertes Glykol hinter der Pumpe 30 aus Leitung 32 über Leitung 31 abgezweigt. Hinter der Glykolpumpe 30 befindet sich das Glykol in Leitung 32 auf hohem Druck, der ausreicht, um das Glykol durch den Kreislauf der Glykolreinigung zu pumpen. Dadurch kann die zusätzliche Pumpe 12 (Fig. 1) entfallen.

Das Glykol gelangt über Leitung 31 zur Mischeinrichtung 13, wo Wasser zugemischt wird. Anschließend wird die Glykol-Wasser-Mischung durch den Anionenaustauscher 21 geführt. Dort kommt es zur Ausflockung von Verunreinigungen, die anschließend im Sandbettfilter 22 herausgefiltert werden. Aus dem Sandbettfilter 22 gelangt die Glykol-Wasser-Mischung in einen Tank 27. Von dort wird sie über Leitung 19 in den Arbeitsbehälter des Reboilers 9 zur Regeneration zurückgeführt.

Der zweite Anionenaustauscher zum Austauschen der Chlorid-lonen gegen Hydroxid-lonen ist zur Vereinfachung in Fig. 2 nicht dargestellt. Außerdem ist das Sandbettfilter 22 nur einstufig dargestellt. Neben dem Sandbettfilter 22 sind in Fig. 2 die Einrichtungen zum Reinigen des Sandbettfilters 22 durch Rückspülen mit einer Glykol-Wasser-Mischung gezeigt. Die zum Reinigen verwendete Glykol-Wasser-Mischung wird aus dem Tank 27 mit Hilfe der Pumpe 28 in umgekehrter Richtung durch das Sandbettfilter 22 geleitet. Die mit den aus dem Sandbettfilter herausgespülten Verunreinigungen beladene Glykol-Wasser-Mischung wird anschließend durch eine Filtereinrichtung 29 geleitet und in den Tank 27 zurückgeführt. Dabei kann die zum Reinigen verwendete Glykol-Wasser-Mischung mehrfach durch diesen Kreislauf geführt werden.

Im Rahmen der Erfindung sind eine Reihe alternativer Ausführungsbeispiele denkbar. Beispielsweise kann der zur Entsalzung verwendete zweite Anionenaustauscher auch zwischen dem ersten Anionenaustauscher und der Filtereinrichtung oder zwischen zwei Stufen der Filtereinrichtung angeordnet sein. Darüber hinaus kann das Reinigungsverfahren auch im Hauptstrom der Regenerierung eingebunden sein, was allerdings nur bei geringen Mengen und starker Verunreinigung sinnvoll sein dürfte.

## Patentansprüche

1. Verfahren zum Trocknen eines Gases, insbesondere von Erdgas, unter Verwendung eines Glykols, wobei
- das Gas einem Absorber zugeführt wird,
- in dem Absorber das Gas in innigen Kontakt mit dem Glykol gebracht wird, wobei das Glykol zumindest einen Teil der Feuchtigkeit aus dem Gas und Verunreinigungen aufnimmt,
- das Gas aus dem Absorber abgeführt wird; das wasserbeladene, verunreinigte Glykol aus dem Absorber abgeführt und regeneriert wird, indem das Wasser durch Erhitzen ausgetrieben wird, wobei wenigstens ein Teil des aus dem Absorber abgeführten Glykols gereinigt wird, indem das verunreinigte Glykol mit mindestens der halben Menge Wasser gemischt wird, die Glykol-Wasser-Mischung auf eine Temperatur oberhalb ihres Trübungspunktes gebracht und dort für eine vorgegebene Zeitdauer gehalten wird, wobei Verunreinigungen ausflocken, die ausgeflockten Verunreinigungen entfernt werden und das Wasser aus der Glykol-Wasser-Mischung durch Erhitzen ausgetrieben wird,
- und das regenerierte Glykol erneut dem Absorber zugeführt wird.

2. Verfahren nach Anspruch 1, dadurch gekennzeichnet, daß nach Entfernen der ausgeflockten Verunreinigungen die Glykol-Wasser-Mischung erneut mit zu regenerierendem, aus dem Absorber abgeführtem Glykol gemischt und anschließend regeneriert wird.

3. Verfahren nach Anspruch 1 oder 2, dadurch gekennzeichnet, daß zum Enffernen der ausgeflockten Verunreinigungen die Glykol-Wasser-Mischung filtriert und/oder zentrifugiert wird.

4. Verfahren nach einem der Ansprüche 1 bis 3, dadurch gekennzeichnet, daß die Glykol-Wasser-Mischung vor dem Erwärmen über einen ersten Anionenaustauscher geführt wird, wobei aus der Mischung anionische Tenside entfernt werden.

5. Verfahren nach einem der Ansprüche 1 bis 4, dadurch gekennzeichnet, daß beim Reinigen die Glykol-Wasser-Mischung auf eine Temperatur von etwa 40°C bis 90°C gebracht und dort für eine Zeitdauer zwischen 2 und 30 Minuten gehalten wird.

6. Verfahren nach einem der Ansprüche 1 bis 5, dadurch gekennzeichnet, daß Triethylenglykol verwendet wird und daß beim Reinigen das verunreinigte Triethylenglykol mit Wasser etwa im Verhältnis 1:1 gemischt wird, und die Triethylenglykol-Wasser-Mischung auf eine Temperatur von etwa 75°C bis 85°C gebracht und dort für eine Zeitdauer zwischen 5 und 20 Minuten gehalten wird.

7. Verfahren zum Reinigen von in einer Gastrocknungsanlage verunreinigtem Glykol, wobei
- verunreinigtes Glykol aus der Gastrocknungsanlage abgeführt wird,
- das abgeführte Glykol mit mindestens der halben Menge Wasser vermischt wird,
- die Glykol-Wasser-Mischung über einen ersten lonenaustauscher geführt wird, wobei aus der Glykol-Wasser-Mischung anionische Tenside entfernt werden, wobei der Trübungspunkt unter die Temperatur der Glykol-Wasser-Mischung abgesenkt wird, wodurch Verunreinigungen ausflocken,
- die ausgeflockten Verunreinigungen entfernt werden, und
- die gereinigte Glykol-Wasser-Mischung in eine Regenerationsstufe der Gastrocknungsanlage zurückgeführt wird, in der das Wasser ausgetrieben wird.

8. Verfahren nach Anspruch 7, dadurch gekennzeichnet, daß der erste lonenaustauscher mit Anionen einer starken Säure, vorzugsweise mit Chlorid-lonen beladen wurde.

9. Verfahren nach Anspruch 7 oder 8, dadurch gekennzeichnet, daß die gereinigte Glykol-Wasser-Mischung vor dem Zurückführen in die Regenerationsstufe der Gastrocknungsanlage durch einen basischen, mit Hydroxid-lonen beladenen zweiten lonenaustauscher geleitet wird, wobei die im ersten lonenaustauscher der Glykol-Wasser-Mischung zugeführten Anionen zumindest teilweise gegen Hydroxidionen ausgetauscht werden.

10. Verfahren nach einem der Ansprüche 7 bis 9, dadurch gekennzeichnet, daß die Glykol-Wasser-Mischung zur Entfernung der ausgeflockten Verunreinigungen durch ein Sand- oder Kiesbettfilter geführt wird.

11. Anordnung zum Trocknen eines Gases, insbesondere von Erdgas, unter Verwendung eines Glykols, mit
- einem Absorber (1), an dessen einem Ende eine Leitung (2) für das zu trocknende Gas und an dessen gegenüberliegendem Ende eine Leitung (3) für das trockene Gas und eine Leitung (4) für Glykol angeschlossen sind,
- einer Einrichtung (5, 6, 8) zum Abführen wasserbeladenen Glykols aus dem Absorber (1) und zum Zuführen des Glykois zu einem Reboiler (9) zum Regenerieren des Glykols und
- einer Einrichtung (10, 11, 4) zum Rückführen des regenerierten Glykols zum Absorber (1), dadurch gekennzeichnet,
- daß mit der Einrichtung (10, 11, 4) zum Rückführen eine Mischeinrichtung (12, 13, 14) zum Abführen eines Anteils des regenerierten Glykols und zum Zumischen einer vorgegebenen Wassermenge verbunden ist,
- daß mit der Mischeinrichtung (12, 13, 14) eine Absetzkammer (17) verbunden ist,
- daß die Absetzkammer (17) mit einer Trenneinrichtung (18) zum Abtrennen von ausgeflockten Verunreinigungen verbunden ist, und
- daß die Trenneinrichtung (18) mit dem Reboiler (9) verbunden ist.

12. Anordnung nach Anspruch 11, dadurch gekennzeichnet, daß zwischen der Mischeinrichtung (12, 13, 14) und der Absetzkammer (17) ein Anionenaustauscher (16) zur Vorreinigung der Glykol-Wasser-Mischung eingebunden ist.

13. Anordnung nach Anspruch 11 oder 12, dadurch gekennzeichnet, daß zwischen der Mischeinrichtung (12,13,14) und der Trenneinrichtung (18) ein Anionenaustauscher eingebunden ist, in dem aus der Glykol-Wasser-Mischung anionische Tenside entfernt werden.

14. Anordnung zum Reinigen von in einer Gastrocknungsanlage (1-11) verunreinigtem Glykol, wobei die Gastrocknungsanlage (1-11) eine Regenerationsstufe (9) zum Austreiben des Wassers aus einer Glykol-Wasser-Mischung beinhaltet, mit
- einer Mischeinrichtung (13) zum Mischen von aus der Gastrocknungsantage (1-11) abgeführtem Glykol mit Wasser zu einer Glykol-Wasser-Mischung,
- einem mit der Mischeinrichtung (13) verbundenen ersten lonenaustauscher (21) zum Entfernen anionischer Tenside aus der Glykol-Wasser-Mischung,
- einer dem ersten lonenaustauscher (21) nachgeschaltete Filtereinrichtung (22-25) zum Entfernen ausgeflockter Verunreinigungen aus der Glykol-Wasser-Mischung, die über eine Leitung (19) mit der Regenerationsstufe (9) verbunden ist.

15. Anordnung nach Anspruch 14, dadurch gekennzeichnet, daß der erste Ionenaustauscher (21) mit Anionen einer starken Säure, vorzugsweise mit Chlorid-lonen, beladen ist.

16. Anordnung nach Anspruch 14 oder 15, dadurch gekennzeichnet, daß der Filtereinrichtung (22-25) ein basischer, mit Hydroxid-lonen beladener zweiter lonenaustauscher (26) zum Austauschen zumindest eines Teils der im ersten Anionenaustauscher (21) der Glykol-Wasser-Mischung zugeführten Anionen gegen Hydroxid-lonen nachgeordnet ist.

17. Anordnung nach einem der Ansprüche 14 bis 16, dadurch gekennzeichnet, daß die Mischeinrichtung (13) über eine Leitung (31, 32) derart mit einer das Glykol einem Absorber (1) zuführenden Pumpe (30) der Gastrocknungsanlage (1-11) verbunden ist, daß in die Mischeinrichtung (13) ein Teil des aus der Pumpe (30) austretenden Glykols gedrückt wird.

## Claims

1. Method for drying a gas, in particular natural gas, using a glycol, wherein
- the gas is fed to an absorber,
- the gas is brought into contact with the glycol in the absorber, the glycol absorbing at least part of the moisture from the gas and impurities,
- the gas is removed from the absorber; the contaminated glycol laden with water is removed from the absorber and regenerated by heating to drive the water out, at least part of the glycol removed from the absorber being purified by mixing the contaminated glycol with at least half the amount of water, bringing the glycol-water mixture to a temperature above its cloud point and keeping it at said temperature for a predetermined time, during which impurities flocculate, the flocculated impurities are removed and the water is driven out of the glycol-water mixture by heating,
- and the regenerated glycol is returned to the absorber.

2. Method according to claim 1 wherein, after removal of the flocculated impurities, the glycol-water mixture is mixed again and regenerated with the glycol which has been removed from the absorber and is to be regenerated.

3. Method according to claim 1 or 2 wherein the glycol-water mixture is filtered and/or centrifuged to remove the flocculated impurities.

4. Method according to any one of claims 1 through 3 wherein the glycol-water mixture is passed over a first anion exchanger before heating, the anionic surfactants being removed from the mixture.

5. Method according to any one of claims 1 through 4 wherein, during the purification process, the glycol-water mixture is brought to a temperature of approx. 40°C to 90°C and kept at that temperature for a period of between 2 and 30 minutes.

6. Method according to any one of claims 1 through 5 wherein triethylene glycol is used and wherein, during the purification process, the contaminated triethylene glycol is mixed with water in a ratio of roughly 1:1 and the triethylene glycol-water mixture is brought to a temperature of approx. 75°C to 85°C and kept at said temperature for a period of between 5 and 20 minutes.

7. Method for cleaning glycol contaminated in a gas drying plant, wherein
- contaminated glycol is removed from the gas drying plant,
- the glycol removed is mixed with at least half the amount of water,
- the glycol-water mixture is passed over a first ion exchanger, during which anionic surfactants are removed from the glycol-water mixture, the cloud point is reduced below the temperature of the glycol-water mixture thus leading to flocculation of the impurities,
- the flocculated impurities are removed, and
- the purified glycol-water mixture is returned to a regeneration stage of the gas drying plant in which the water is driven out.

8. Method according to claim 7 wherein the first ion exchanger is filled with anions of a strong acid, preferably chloride ions.

9. Method according to claim 7 or 8 wherein the purified glycol-water mixture is passed, before returning to the regeneration stage of the gas drying plant, through a basic second ion exchanger filled with hydroxide ions, the anions fed to the glycol-water mixture in the first ion exchanger being replaced at least partially by hydroxide ions.

10. Method according to any one of claims 7 through 9 wherein the glycol-water mixture is passed through a sand or gravel filter to remove the flocculated impurities.

11. Arrangement for drying a gas, in particular natural gas, using a glycol, with
- an absorber (1), on one end of which a line (2) is connected for the gas to be dried and on the other end of which a line (3) for the dry gas and a line (4) for glycol are connected,
- a device (5, 6, 8) for removing glycol laden with water from the absorber (1) and for feeding the glycol to a reboiler (9) to regenerate the glycol and
- a device (10, 11, 4) for returning the regenerated glycol to the absorber (1)
- wherein a mixing device (12, 13, 14) for removing part of the regenerated glycol and for adding a predetermined amount of water is connected to the device (10, 11, 4) for returning the regenerated glycol to the absorber (1),
- wherein the mixing device (12, 13, 14) is connected to a settling chamber (17)
- wherein the settling chamber (17) is connected to a separating device (18) for separating flocculated impurities, and
- wherein the separating device (18) is connected to the reboiler (9).

12. Arrangement according to claim 11 wherein between the mixing device (12, 13, 14) and the settling chamber (17) an anion exchanger (16) is integrated to preclean the glycol-water mixture.

13. Arrangement according to claim 11 or 12 wherein between the mixing device (12, 13, 14) and the separating device (18) an anion exchanger is integrated in which anionic surfactants are removed from the glycol-water mixture.

14. Arrangement for purifying glycol contaminated in a gas drying plant (1-11), wherein the gas drying plant (1-11) contains a regeneration stage (9) for driving out the water from a glycol-water mixture, with
- a mixing device (13) for mixing glycol removed from the gas drying plant (1-11) with water to form a glycol-water mixture,
- a first ion exchanger (21) for removing anionic surfactants from the glycol-water mixture and connected to the mixing device (13),
- a filter device (22-25) for removing flocculated impurities from the glycol-water mixture arranged downstream of the first ion exchanger (21) and connected via a line (19) to the regeneration stage (9).

15. Arrangement according to claim 14 wherein the first ion exchanger (21) is filled with anions of a strong acid, preferably with chloride ions.

16. Arrangement according to claim 14 or 15 wherein a basic second ion exchanger (26) filled with hydroxide ions for replacing at least part of the anions fed to the glycol-water mixture in the first anion exchanger (21) by hydroxide ions is arranged downstream of the filter device (22-25).

17. Arrangement according to any one of claims 14 through 16 wherein the mixing device (13) is connected via a line (31, 32) in such a way with a pump (30) of the gas drying plant (1-11) feeding the glycol to an absorber (1) that part of the glycol leaving the pump (30) is pressed into the mixing device (13).

## Revendications

1. Procédé pour le séchage d'un gaz, en particulier de gaz naturel, en utilisant du glycol, où
- le gaz est acheminé vers un absorbeur;
- dans l'absorbeur, le gaz est mis en contact étroit avec le glycol, ce dernier absorbant au moins une partie de l'humidité et des impuretés du gaz;
- le gaz est évacué de l'absorbeur; le glycol chargé d'eau et d'impuretés est évacué de l'absorbeur et régénéré en ce que l'eau est chassée par échauffement, par où au moins une partie du glycol évacué de l'absorbeur est nettoyé, en ce que le glycol chargé d'impuretés est mélangé avec au moins pour la moitié d'eau, le mélange glycol/eau est mis à une température au-dessus de son point de trouble et maintenu à cette température pendant une durée prédéterminée, par où des impuretés floculent, en ce que les impuretés ayant floculé sont éliminés et l'eau du mélange glycol/eau est chassée par échauffement; et
- le glycol régénéré est réacheminé vers l'absorbeur.

2. Procédé suivant la revendication 1, caractérisé par le fait que, après élimination des impuretés ayant floculé, le mélange glycol/eau est de nouveau mélangé avec du glycol à régénérer, issu de l'absorbeur, et ensuite régénéré.

3. Procédé suivant l'une des revendications 1 ou 2, caractérisé par le fait que pour l'élimination des impuretés ayant floculé, le mélange glycol/eau et filtré et/ou centrifugé.

4. Procédé suivant l'une des revendications 1 à 3, caractérisé par le fait que le mélange glycol/eau est conduit, avant l'échauffement, sur un premier échangeur d'anions, par où des agents tensioactifs anioniques sont éliminés du mélange.

5. Procédé suivant l'une des revendications 1 à 4, caractérisé par le fait que lors du nettoyage, le mélange glycol/eau est mis à une température d'environ 40°C à 90°C et maintenu à cette température pendant une période comprise entre 2 et 30 minutes.

6. Procédé suivant l'une des revendications 1 à 5, caractérisé par le fait que du triéthylèneglycol est utilisé et que lors du nettoyage, le triéthylèneglycol chargé d'impuretés est mélangé avec de l'eau à concurrence de 1:1 environ et que le mélange triéthylèneglycol/eau est mis à une température d'environ 75°C à 85°C et maintenu à cette température pendant une période comprise entre 5 et 20 minutes.

7. Procédé pour le nettoyage de glycol, chargé d'impuretés dans une installation de séchage de gaz, où
- le glycol chargé d'impuretés est évacué de l'installation de séchage;
- le glycol évacué est mélangé avec au moins pour la moitié d'eau;
- le mélange glycol/eau est conduit sur un premier échangeur d'ions, par où des agents tensioactifs anioniques sont éliminés du mélange glycol/eau, par où le point de trouble est abaissé en dessous de la température du mélange glycol/eau, par où des impuretés floculent;
- les impuretés ayant floculé sont éliminés; et
- le mélange glycol/eau nettoyé est acheminé vers une cellule de régénération de l'installation de séchage, dans laquelle l'eau est chassée.

8. Procédé suivant la revendication 7, caractérisé par le fait que le premier échangeur d'ions a été chargé d'anions d'un acide fort, de préférence d'ions de chlorure.

9. Procédé suivant l'une des revendications 7 ou 8, caractérisé par le fait que le mélange glycol/eau nettoyé est conduit, avant d'être réacheminé vers la cellule de régénération de l'installation de séchage, sur un deuxième échangeur d'ions basique, chargé d'ions d'hydroxyde, par où les anions amené au mélange glycol/eau dans le premier échangeur d'ions sont au moins en partie échangés contre des ions d'hydroxyde.

10. Procédé suivant l'une des revendications 7 à 9, caractérisé par le fait que le mélange glycol/eau est conduit à travers un filtre à couche de sable ou de gravier pour l'enlèvement des impuretés ayant floculé.

11. Dispositif pour le séchage d'un gaz, en particulier de gaz naturel, en utilisant du glycol, avec
- un adsorbeur (1), à l'une des extrémités duquel est raccordée une conduite (2) pour le gaz à sécher et à l'autre extrémité duquel sont raccordées une conduite (3) pour le gaz sec et une conduite (4) pour du glycol;
- un dispositif (5, 6, 8) pour l'évacuation du glycol chargé d'eau de l'adsorbeur (1) et pour l'acheminement du glycol vers un élément chauffant (9) pour la régénération du glycol; et
- un dispositif (10, 11, 4) pour le réacheminement du glycol régénéré vers l'adsorbeur (1), caractérisé par le fait
- qu'au dispositif (10, 11, 4) pour le réacheminement est lié un dispositif de mélange (12, 13, 14) pour l'évacuation d'une partie du glycol régénéré et l'ajout d'une quantité prédéterminée d'eau;
- qu'au dispositif de mélange (12, 13, 14) est lié une chambre de dépôt (17);
- que la chambre de dépôt (17) et liée à un dispositif de séparation (18) pour la séparation d'impuretés ayant floculé; et
- que le dispositif de séparation (18) est lié à l'élément chauffant (9).

12. Dispositif suivant la revendication 11, caractérisé par le fait que, entre le dispositif de mélange (12, 13, 14) et la chambre de dépôt (17), est intégré un échangeur d'anions (16) pour le prénettoyage du mélange glycol/eau.

13. Dispositif suivant l'une des revendications 11 ou 12, caractérisé par le fait que, entre le dispositif de mélange (12, 13, 14) et le dispositif de séparation (18), est intégré un échangeur d'anions, dans lequel des agents tensioactifs anioniques sont éliminés du mélange glycol/eau.

14. Dispositif pour le nettoyage de glycol, chargé d'impuretés dans une installation de séchage de gaz (1-11), l'installation de séchage de gaz (1-11) comportant une cellule de régénération (9) pour chasser l'eau du mélange glycol/eau, avec
- un dispositif de mélange (13) pour mélanger du glycol évacué de l'installation de séchage de gaz (1-11) avec de l'eau pour former un mélange glycol/eau;
- un premier échangeur d'ions (21), lié au dispositif de mélange, pour éliminer des agents tensioactifs anioniques du mélange glycol/eau;
- un dispositif de filtrage (22-25), disposé en aval du premier échangeur d'ions (21), pour la séparation d'impuretés ayant floculé du mélange glycol/eau et qui est lié à la cellule de régénération (9) par une conduite (19).

15. Dispositif suivant la revendication 14, caractérisé par le fait que le premier échangeur d'ions (21) est chargé d'anions d'un acide fort, de préférence d'ions de chlorure.

16. Dispositif suivant l'une des revendications 14 ou 15, caractérisé par le fait qu'à l'aval du dispositif de filtrage (22-25) est disposé un deuxième échangeur d'ions basique (26), chargé d'ions d'hydroxyde, pour échanger au moins une partie des anions amené au mélange glycol/eau dans le premier échangeur d'anions (21) contre des ions d'hydroxyde.

17. Dispositif suivant l'une des revendications 14 à 16, caractérisé par le fait que le dispositif de mélange (13) est lié, moyennant une conduite (31, 32), à une pompe (30) de l'installation de séchage de gaz (1-11) acheminant le glycol à l'adsorbeur (1) de la sorte qu'une partie du glycol sortant de la pompe (30) est poussée dans le dispositif de mélange (13).
